# EUROPEAN PATENT SPECIFICATION

(11) **EP 4 622 573 B1**
(45) Date of publication and mention of the grant of the patent: **04.11.2026**
(21) Application number: 23805965.3
(22) Date of filing: 14.11.2023
(51) Int. Cl.: A61B 18/14, A61B 18/04, A61B 18/18, A61B 18/00, A61B 18/12

(54) **ELECTROSURGICAL INSTRUMENT**
ELEKTROCHIRURGISCHES INSTRUMENT
INSTRUMENT ÉLECTRO-CHIRURGICAL

(30) Priority: 21.11.2022 GB 202217386
(43) Date of publication of application: 01.10.2025
(73) Proprietor: Creo Medical Ltd, Chepstow, South Wales NP16 5UH (GB)
(72) Inventor: HANCOCK, Christopher Paul, Chepstow South Wales NP16 5UH (GB); ULLRICH, George Christian, Chepstow South Wales NP16 5UH (GB); THOMAS, Steven, Chepstow South Wales NP16 5UH (GB); DAWE, Thomas, Chepstow NP16 5UH (GB)
(74) Representative: Mewburn Ellis LLP
(86) International application number: PCT/EP2023/081782
(87) International publication number: WO 2024/110259

(56) References cited:
- WO-A1-03/055402
- US-A1- 2015 196 353
- US-A1- 2016 324 576
- US-A1- 2017 189 114
- US-B2- 10 251 697

## Description

### Field of the Invention

The present invention relates to an electrosurgical instrument. The electrosurgical instrument has an instrument tip which may be configured for delivering electromagnetic energy (e.g. radiofrequency and/or microwave frequency energy) into biological tissue for cutting tissue and/or for haemostasis (i.e. promoting blood coagulation). For example, the instrument may be sized to be suitable for insertion through the instrument channel of a standard surgical endoscope.

### Background

Surgical resection is a means of removing sections of organs from within the human or animal body. Such organs may be highly vascular. When tissue is cut (divided or transected) small blood vessels called arterioles are damaged or ruptured. Initial bleeding is followed by a coagulation cascade where the blood is turned into a clot in an attempt to plug the bleeding point. During an operation, it is desirable for a patient to lose as little blood as possible, so various devices have been developed in an attempt to provide blood free cutting. For endoscopic procedures, bleeds are also undesirable, and need to be dealt with in an expedient manner, since the blood flow may obscure the operator's vision, which may prolong surgery and potentially lead to the procedure needing to be terminated and another method used instead, e.g. open surgery.

Electrosurgical generators are prevalent in hospital operating theatres, often for use in open and laparoscopic procedures, and increasingly for use with surgical scoping devices, e.g. an endoscope or the like. In endoscopic procedures the electrosurgical accessory is typically inserted through a lumen inside an endoscope. Considered against the equivalent access channel for laparoscopic surgery, such a lumen is comparatively narrow in bore and greater in length.

It is known to use microwave frequency energy for haemostasis (i.e. sealing broken blood vessels by promoting blood coagulation). Instruments are known that radiate microwave energy from the edges of a planar transmission line to cause localised tissue ablation or coagulation.

Additionally, instead of a sharp blade, it is known to use radiofrequency (RF) energy to cut biological tissue. The method of cutting using RF energy operates using the principle that as an electric current passes through a tissue matrix (aided by the ionic contents of the cells and the intercellular electrolytes), the impedance to the flow of electrons across the tissue generates heat. In practice, an instrument is arranged to apply an RF voltage across the tissue matrix that is sufficient to generate heat within the cells to vaporise the water content of the tissue. However, as a result of this increasing desiccation, particularly adjacent to the RF emitting region of the instrument (which has the highest current density of the current path through tissue), direct physical contact between the tissue and instrument can be lost. The applied voltage then manifests itself as a voltage drop across this small void, which causes ionisation in the void that leads to a plasma. Plasma has a very high volume resistivity compared with tissue. The energy supplied to the instrument maintains the plasma, i.e. completes the electrical circuit between the instrument and the tissue. Volatile material entering the plasma can be vaporised and the perception is therefore of a tissue dissecting plasma.

GB 2 523 246 describes an electrosurgical instrument for applying to biological tissue RF electromagnetic energy and/or microwave frequency EM energy. The instrument comprises a shaft insertable through an instrument channel of a surgical scoping device. At a distal end of the shaft there is an instrument tip comprising a planar transmission line formed from a sheet of a first dielectric material having first and second conductive layers on opposite surfaces thereof. The planar transmission line is connected to a coaxial cable conveyed by the shaft. The coaxial cable is arranged to deliver either microwave or RF energy to the planar transmission line. The coaxial cable comprises an inner conductor, an outer conductor coaxial with the inner conductor, and a second dielectric material separating the outer and inner conductors, the inner and outer conductors extending beyond the second dielectric at a connection interface to overlap opposite surfaces of the transmission line and electrically contact the first conductive layer and second conductive layer respectively. The instrument further comprises a protective hull with a smoothly contoured convex undersurface facing away from the planar transmission line. The undersurface comprises a longitudinally extending recessed channel formed therein. A retractable needle is mounted within the instrument, and operable to extend through the recessed channel to protrude from a distal end of the instrument. The needle can be used to inject fluid into a treatment zone before the RF or microwave energy is applied.

US20170189114A1 discloses an electrosurgical instrument with dual radiofrequency and microwave electromagnetic energy.

The present invention has been devised in light of the above considerations.

### Summary of the Invention

The present invention provides a development to the concept discussed in GB 2 523 246.

The invention is defined by the appended independent claim. Optional features of the invention are defined by the appended dependent claims.

At its most general, the inventors have developed a modified instrument tip that is shaped for improved positional control.

According to the first aspect of the disclosure, there is provided an electrosurgical instrument for applying radiofrequency (RF) electromagnetic (EM) energy and/or microwave frequency EM energy to biological tissue, the electrosurgical instrument comprising: an instrument tip comprising a planar body made of a first dielectric material separating a first conductive element on a first surface thereof from a second conductive element on a second surface thereof, the second surface facing in the opposite direction to the first surface; a coaxial feed cable comprising an inner conductor, an outer conductor coaxial with the inner conductor and a dielectric material separating the inner and outer conductors, the coaxial feed cable being for conveying an RF signal and/or microwave signal; wherein the inner conductor is electrically connected to the first conductive element and the outer conductor is electrically connected to the second conductive element to enable the instrument tip to receive the RF signal and/or the microwave signal, wherein an edge of the instrument tip has a notch defining a recess for engaging tissue within the recess.

This arrangement may advantageously help to provide improved control of the instrument tip and to reduce the risk of accidental perforations. In particular, by engaging tissue within the recess, the notch may help to increase traction of the instrument tip within tissue, thereby helping to prevent slipping of the instrument tip. The notch may be configured in various manners to provide different engagement characteristics with tissue, in order to suit different surgical applications and/or to individual clinician preferences. Further, the location of the notch in the edge of the instrument tip may act as a visual indicator of insertion depth (e.g. lateral or axial insertion depth) into biological tissue. This may improve ease of use, helping a clinician to become more easily accustomed to using the instrument, particularly if it has different (e.g. smaller) dimensions from an instrument previously used by the clinician.

As used herein, the term "edge" with reference to the instrument tip may refer to any region extending around the periphery of the instrument tip, including at a distal and/or lateral region(s) thereof. Accordingly, the notch may be located at any suitable edge for engaging tissue, e.g. along a distal edge and/or lateral edge(s) of the instrument tip.

As used herein, the term "notch" may refer to an indentation or cut-out that forms a discontinuity (e.g. corner, bend, inflection, crook) in a surface at the edge (periphery) of the instrument tip. This defines a recess for engaging (e.g. snagging, hooking, catching, dragging) tissue. In order to engage tissue, the notch is preferably located at an exposed portion of the instrument tip that is located distally of a flexible shaft which connects the coaxial feed cable to the instrument tip.

The notch may be considered to comprise a rear wall and one or more side walls which define the recess. The rear wall and/or side walls may blend together smoothly (thereby omitting a sharp corner therebetween, so that there may be no distinctly visible boundary between the rear and side walls), or may be joined e.g. at a sharp bend or corner to clearly delimit the respective walls. In this context, the term "rear wall" may refer to a surface of the notch that is located laterally *inwardly* with respect to the surfaces of the edge that are adjacent the notch, e.g. with the rear wall being located nearer to a central longitudinal axis of the instrument tip if the notch is in a lateral edge of the instrument tip, or with the rear wall being the most proximal wall of the notch if the notch is in a distal edge of the instrument tip. The term "side wall" may refer to a surface that defines an end region of the notch.

The notch may be formed in any suitable element of the instrument tip for contacting tissue. Preferably, the notch may be formed in the planar body of the instrument tip. Optionally, the notch may also be formed in the first and/or second conductive elements, which may extend up to an edge (e.g. distal edge) of the planar body. Preferably, the first and/or second conductive elements extend up to the edge of the notch, for treating tissue that is engaged within the recess of the notch. In some embodiments, the instrument tip may include a protective hull, which may include the notch formed in an edge thereof. The protective hull may comprise a piece of dielectric material mounted to cover the underside of the planar body. The protective hull may have a smoothly contoured convex undersurface facing away from the planar body.

As mentioned above, different notch configurations may be desirable depending on surgeon preferences and/or depending on the procedure to be performed. For example, optionally, at least one end of the notch forms a pointed corner in the edge of the instrument tip. As used herein, the term "pointed corner" refers to a junction between two surfaces of the instrument tip, which are angled to form a distinct and clearly visible boundary/point. This may be contrasted from two edges which are smoothly contoured together, with no clear boundary therebetween. The two surfaces/walls which meet to form the pointed corner may be planar and/or curved. For simplicity, a "pointed corner" may also alternatively be referred to herein as a "corner". Advantageously, providing a notch that forms a pointed corner may help to catch or snag vessels using the notch. Further, since the corner helps to precisely delimit the ends of the notch, it can help to provide a clearer indication for measuring insertion depth (e.g. compared to a smooth contour).

Optionally, the notch may form a pair of corners, one at each (lateral or axial) end of the notch. This may provide similar advantages as discussed above for grasping/snagging tissue, as well as providing an additional visual reference for measuring (lateral or axial) insertion depth.

The pointed corner may form an angle which is acute, perpendicular, or obtuse (as measured between the two adjoining surfaces of the instrument tip, in a direction through the instrument tip).

An obtuse corner may be particularly useful for applications benefiting from relatively smooth insertion/retraction, while still providing a distinct point for improved perception of insertion depth compared to e.g. a smoothly contoured end region. Further, an obtuse corner may permit easier access to the recess and thereby facilitate easier cleaning, compared to an acute or perpendicular corner.

An acute corner may be particularly useful for snagging/catching individual tissue fibres, and for preventing slipping of the instrument tip when the instrument tip is pulled/pushed against the acute corner. Conversely, when the instrument tip is pulled/pushed in the opposite direction, the acute corner may facilitate smooth insertion/retraction of the instrument tip.

Optionally, the pointed corner may comprise a barb pointing in a proximal direction. As used herein, the term "barb" takes its common meaning relating to a projection which is angled away from a main (distal) point so as to make extraction more difficult. Accordingly, the barb relates to a type of acute corner that points towards a proximal direction (i.e. away from a distal direction). Optionally, the barb may also point in a lateral (left/right) direction, so as to be oriented at a non-parallel angle relative to a longitudinal axis of the instrument tip.

An arrangement having a barb may be particularly advantageous for enabling smooth insertion of the instrument tip (since the barb points away from the distal end and so will not snag on fibres when moving distally) while also enabling snagging/pulling against individual tissue fibres when the instrument tip is pulled proximally.

The corner (e.g. barb) may have a dull/smooth or sharpened tip. A sharp tip may help to facilitate cutting of tissue/vessels.

Alternatively, the pointed corner may point in a distal direction, in which case it may be referred to as a "piercing element". Preferably, the piercing element may be located at a distal end of the instrument tip. This allows the piercing element to impinge upon tissue and improve the distal cutting properties of the instrument tip, as will be discussed further below.

Alternatively, as noted above, a pointed corner may be substantially perpendicular rather than acute or obtuse. A substantially perpendicular corner may provide a useful balance between the above acute and obtuse arrangements, helping to snag tissue without significantly inhibiting smooth proximal and distal insertion. Further, a perpendicular corner may facilitate easier cleaning compared to an acute (e.g. barbed) corner.

Optionally, a distal end (e.g. only a distal end) of the notch forms the pointed corner. This may enable the distal end of the notch to be used for detailed work, such as snagging / catching of tissue near the distal end of the instrument tip.

In arrangements where the notch is located at a distal edge (e.g. distal end face) of the instrument tip and has a pair of lateral (left and right) walls, each lateral wall may be considered to terminate in a respective "distal end point". Accordingly, in some embodiments, the notch may have a pair of distal end points, one or more of which may form a respective pointed corner.

Alternatively, in arrangements where the notch is located along a lateral edge of the instrument tip, the notch may comprise both a proximal end (near a proximal end of the instrument tip) and a distal end (near a distal end of the instrument tip), where the distal end may form the pointed corner. Alternatively, in some embodiments, the proximal end (e.g. only the proximal end) of the notch may form the pointed corner.

Optionally, both ends of the notch may form a respective pointed corner in the edge of the instrument tip. The pointed corners at each end may have similar/complementary configurations or may have different configurations, depending on the desired application and surgeon preferences.

Optionally, at least one end of the notch may be smoothly contoured into the edge of the instrument tip (e.g. at one or both end regions of the notch). As used herein, the term "smoothly contoured" may refer to a curved region in the edge of the instrument tip, with an inflection point where the edge changes curvature in order to smoothly define an end region of the notch, without forming a distinct corner.

A smooth contour may be particularly advantageous for providing smooth insertion/retraction, while also providing increased traction/friction due to the increased surface area of the notch, thereby helping to prevent the instrument tip from slipping relative to the tissue. Additionally, a smooth contour may help to further facilitate ease of cleaning compared to a pointed (e.g. acute, barbed) corner.

**It** will be noted that the notches may be referred to herein as having a first and/or second "end". For example, these may be a first axial end and second axial end (e.g. a distal end and proximal end); or a first lateral end and second lateral end (e.g. a left end and right end, which may each be located at a distal end of the instrument tip). In notches having pointed corners at both ends, the ends will be abrupt and distinctly visible. However, embodiments where the notch is smoothly contoured into the edge of the instrument tip at one or both ends, its end(s) may be less abrupt/distinct. Accordingly, references to a "first end" or "second end" may interchangeably be referred to as a first end *region* and a second end *region.*

Optionally, both ends of the notch may be smoothly contoured into the edge of the instrument tip.

Optionally, a proximal end (e.g. only a proximal end) of the notch may be smoothly contoured into the edge of the instrument. This may help to provide smooth insertion and retraction. Optionally, a distal end of the notch may form a pointed corner (e.g. barb, obtuse corner, or perpendicular corner) as discussed above. Providing a smooth contour in combination with a pointed corner may help to facilitate smooth insertion / retraction (via the smooth contour) while also providing the advantages of improved grasping and insertion depth measurement at the distal end (via the pointed corner).

Optionally, the notch may be located at a distal end of the instrument tip and may face distally away from the instrument tip. By providing a distally-facing notch, the instrument tip may have better performance/traction when delivering energy end-on to a vessel/tissue. The notch may be shaped in various manners to provide desired characteristics against vessels/tissue. For example, the notch may form a pair of pointed corners defining the recess for engaging (receiving) tissue therebetween, each corner facing away from the distal end of the instrument tip to impinge upon tissue. This may be particularly useful for grasping individual fibres/vessels at a distal end of the instrument, within a cavity defined by the notch. A distally-facing notch may also be referred to as a "fish mouth" type notch.

Optionally, one or both pointed corners may be configured as a piercing element defining an acute angle pointing in a distal direction, as discussed above. Optionally, the piercing element(s) may also point inwardly towards a central axis of the instrument tip. This may help to retain/grasp a vessel within the recess.

The notch may be configured in various manners to define different types of recesses. For example, optionally, the notch may define a smoothly contoured concave recess (cavity) for grasping vessels/fibres. Alternatively, the notch may comprise one or more corners/bends to delineate a respective two or more walls (e.g. planar walls). For example, the notch may define a triangular recess formed by a pair of angled walls. Alternatively, the notch may comprise three walls, which may provide a substantially rectangular or trapezoidal recess. The shape of the recess may be selected based on vessel characteristics and desired interaction with tissue.

Optionally, the notch may define a hook-shaped recess for catching/snagging/hooking tissue within the recess. In such embodiments, the hook may terminate in a barb as discussed above.

As discussed above, optionally, the notch may be located at a lateral edge of the instrument tip. Locating the notch at the lateral edge may be particularly advantageous for providing depth perception, and/or for applications where side-on (rather than end-on) cutting/coagulation is desired.

Optionally, the notch may be an elongate notch (e.g. a single elongate notch) located along the lateral edge of the instrument tip. The term "elongate" may refer to a notch which is longer (from a proximal end to a distal end) than it is deep (measured as a lateral depth of the recess into the instrument tip, e.g. perpendicular to the edge of the instrument tip). Advantageously, an elongate notch may facilitate easy cleaning, since it is less likely to get clogged with biological matter compared to short notches.

As used herein, the term "lateral depth" may refer to a depth of the notch as measured into the edge of the instrument (e.g. perpendicular to the axial direction). Conversely, the term "axial depth" or "insertion depth" may refer to a depth as measured along the length of the instrument tip, i.e. in an axial direction.

The length of the elongate notch (from a first end to the second end) may provide an indication of insertion depth. For example, the elongate notch may have a length of approximately 1mm. Optionally, the elongate notch may form a corner in the edge of the planar body, which may further facilitate this visual indication. The corner may be configured in any suitable manner, as discussed above.

In variant embodiments, the elongate notch may be smoothly contoured into the lateral side of the instrument tip.

Optionally, the notch has a lateral depth (measured into the instrument tip) of less than 30% of the maximum width of the instrument tip, optionally less than 20%, optionally less than 15%. The depth of the notch may be varied to provide smoother insertion and easy cleaning (by providing a shallower notch at e.g. ≤15% of the instrument tip's maximum width) or improved grasping/traction (by providing a deeper notch at e.g. >15% of the instrument tip's maximum width).

Optionally, the instrument tip has two or more notches. Each notch may be configured in any suitable manner as discussed herein. The notches may have the same/similar or different configurations (e.g. both elongate notches, or one short notch and one long notch). Optionally, the two or more notches may be spaced apart in an axial direction. The axial spacing of the plurality of notches may help to provide an indication of insertion depth. One or more of the notches may be "short" notches, e.g. having a depth substantially equal to their width. Optionally, the short notch(es) may be smoothly contoured into the edge of the instrument tip, in order to improve ease of cleaning and reduce the chance of becoming clogged (which short notches may be particularly susceptible to, e.g. compared to elongate notches). Optionally, the two or more notches may be located and axially spaced along the same ("first") lateral side of the instrument tip, which may further help to measure the insertion depth.

Optionally, the two or more notches may be located at opposite lateral edges of the instrument tip. Both sides of the instrument tip may therefore be used to provide cutting/snagging of tissue. Optionally, the notches may be axially aligned. This may provide a similar function on both sides of the device, thereby facilitating ease of use. Optionally, the two or more notches may together form an elongate thinned section of the instrument tip, the thinned section having a width of less than 50% of the maximum width of the instrument tip, optionally less than 40%, optionally less than 30%, optionally less than 20%. The term "elongate" refers to the section being longer (in an axial direction) than it is wide (in a lateral direction). The thinned section may provide several advantages, as set out below.

In some embodiments, the elongate thinned section terminates in a rounded end. The rounded end may form an end portion of the instrument tip. In such embodiments, the instrument tip may also be referred to as having a "pen tip" configuration. In these embodiments, the thinned section may advantageously allow precise and detailed cutting, and may be easily cleaned since the instrument tip terminates with the elongate thinned section and rounded end rather than including a further enlarged section distally of the elongate thinned section.

In alternative embodiments, the elongate thinned section may terminate in an enlarged section, which may define a pointed corner (e.g. barb). In such embodiments, the thinned section may advantageously increase the amount of tissue that can be caught within the notch, proximally to the pointed corner. In embodiments where the pointed corner is configured as a barb, the instrument tip may be referred to as having a "barbed tip".

Optionally the instrument tip may be substantially symmetrical across its central axis. Thus, the instrument tip may have the same notch configuration on both lateral sides. This may facilitate ease of use.

Optionally, the instrument tip may have a different notch configuration on a first lateral side of the instrument tip than on a second lateral side of the instrument tip. This may allow different functions to be performed depending on the orientation of the device, e.g. to interface with different types of vessels, to provide varying degrees of snagging, to provide different types of cuts, and/or to help reduce slipping.

For example, the notches may be axially spaced apart along the opposite lateral sides of the instrument tip, e.g. in a staggered arrangement. This may allow different types of cuts to be performed along the first side of the instrument tip compared to the second side, depending on the orientation of the device.

Alternatively, for example, the instrument tip may have one or more notches on its first lateral side, and may have no notches on its second lateral side. This may facilitate smooth insertion on the second lateral side, while providing a desired function (e.g. snagging, hooking, insertion depth measurement, etc) via the notches on the first lateral side.

Optionally, the instrument tip includes only a single notch. For example, this may be a single notch located at a distal end of the instrument tip (which may be symmetrical). Such an arrangement may be referred to as a fish-mouth notch, for head-on engagement of tissue. Alternatively, the single notch may be located along only a single lateral side of the instrument tip, to provide single-sided tissue grasping.

As discussed above, the notch may help to provide an indication of axial and/or lateral insertion depth. Alternatively or in combination, optionally, the first conductive element may be patterned to provide an indication of axial and/or lateral depth. For example, the first conductive element can be provided on only a portion of the planar body, with the first conductive element varying in (lateral) width along its (axial) length. In this manner, a region where the first conductive element changes width can be used as an indication of axial (end-on) insertion depth of the instrument tip into biological tissue. Correspondingly, lateral variations in the first conductive element can provide an indication of lateral (side-on) insertion depth into biological tissue. Alternatively or in combination, the first conductive element may be etched (e.g. laser etched) to include a scale for indicating the axial and/or lateral insertion depth of the instrument tip into tissue.

The invention includes the combination of the aspects and preferred features described except where such a combination is clearly impermissible or expressly avoided.

### Summary of the Figures

Example embodiments illustrating the principles of the invention will now be discussed with reference to the accompanying figures in which like numerals denote like elements.
**FIG 1** is a schematic view of a complete electrosurgery system in which the present invention may be applied;
**FIG 2** is a perspective view of an embodiment instrument tip having a pair of elongate notches;
**FIG 3** is a perspective view of an embodiment instrument tip having a plurality of short notches;
**FIGs 4A-4C** are top-down views showing variant elongate notches according to embodiments of the invention;
**FIGs 5A-5B** are top-down views showing variant short notches according to embodiments of the invention;
**FIGs 6A-6C** are top-down views showing variant shapes of notches for hooking tissue, according to embodiments of the invention;
**FIGs 7A-7B** are top-down views showing variant embodiment instrument tips having notches that form a thinned section;
**FIGs 8A-8E** are top-down views showing variant instrument tips which have a different notch configuration on a first lateral side compared to a second lateral side, according to embodiments of the invention;
**FIGs 9A-9D** are top-down views showing embodiment instrument tips that have a distally facing notch;
**FIGs 10A-10C** are top-down views showing embodiment instrument tips that have relatively shallow notches;
**FIG 11** is a top-down view showing various embodiment instrument tips having different notch configurations in their planar bodies and/or first conductive elements;
**FIG 12** is a top-down view showing an embodiment instrument tip connected to a flexible shaft;
**FIG 13** is a perspective view of an embodiment instrument tip connected to a flexible shaft, in which the flexible shaft is transparent for illustrative purposes to show the connections between the instrument tip and a coaxial cable within the interior of the flexible shaft; and
**FIG 14** is a perspective view of the instrument tip and the flexible shaft of FIG 13, in which the flexible shaft is opaque to show the exterior surfaces of the shaft.

### Detailed Description of the Invention

Aspects and embodiments of the present invention will now be discussed with reference to the accompanying figures. Further aspects and embodiments will be apparent to those skilled in the art.

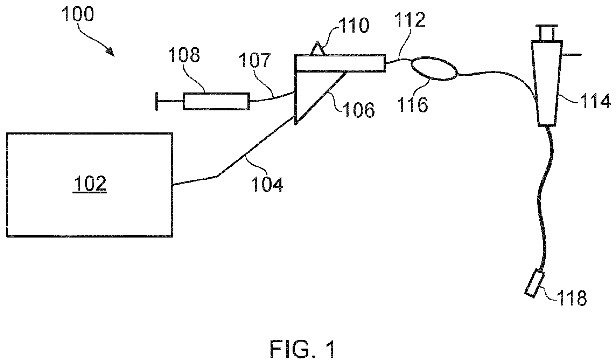

FIG 1 is a schematic diagram of a complete electrosurgery system 100 that is capable of selectively supplying to the distal end of an invasive electrosurgical instrument any or all of RF energy, microwave energy and fluid, e.g. saline or hyaluronic acid. The system 100 comprises a generator 102 for controllable supplying electromagnetic (EM) energy. In the present embodiment, the EM energy includes RF EM energy and/or microwave frequency EM energy. A suitable generator for this purpose is described in WO 2012/076844.

The generator 102 is connected to an interface joint 106 by an interface cable 104. The interface joint 106 is also connected to receive a pressurised fluid supply from a fluid delivery apparatus 108 via a fluid supply cable 107. The function of the interface joint 106 is to combine the inputs from the generator 102 and fluid delivery device 108 into a single flexible shaft 112, which extends from the distal end of the interface joint 106. It is to be understood that the shaft 112 may form part of the interface joint 106.

The flexible shaft 112 is insertable through the entire length of an instrument (working) channel of a surgical scoping device 114. A torque transfer unit 116 may be mounted on a proximal length of the shaft 112 between the interface joint 106 and surgical scoping device 114. The torque transfer unit 116 engages the shaft to permit it to be rotated within the instrument channel of the surgical scoping device 114. The configuration of the torque transfer unit 116 is discussed in more detail below.

The flexible shaft 112 has an electrosurgical instrument tip 118 that is shaped to pass through the instrument channel of the surgical scoping device 114 (e.g. an endoscope) and protrude (e.g. inside the patient) at the distal end of the instrument channel. The instrument tip includes an active tip for delivering RF EM energy and/or microwave EM energy into biological tissue and an aperture for delivering pressurised fluid (e.g. saline, Gelofusine, and/or hyaluronic acid with added marker dye). These combined technologies provide a unique solution for cutting and destroying unwanted tissue and the ability to seal blood vessels around the targeted area. By applying pressure to the fluid, the surgeon is able to inject the fluid between tissues layers in order to distend and mark the position of a lesion to be treated. The injection of fluid in this manner lifts and separates the tissue layers making it both easier to resect around the lesion and plane through the submucosal layer, reducing the risk of bowel wall perforation and unnecessary thermal damage to the muscle layer.

The instrument tip 118 further includes a protective hull positioned under the active tip to assist a tissue planing type resection action, again helping to protect against inadvertent perforation and ensure viability of the remaining tissue, which in turn facilitates more rapid healing and post operation recovery.

The structure of the instrument tip 118 may be particularly designed for use with a conventional steerable flexible endoscope having a working channel with an internal diameters of at least 2.2 mm and a working length of between 60 cm and 170 cm. As such the majority of the comparatively small diameter instrument is housed within the lumen of a much larger and predominantly polymer insulating device, i.e. the flexible endoscope channel. In practice, only 5 mm to 25 mm of the distal assembly protrudes from the distal end of the endoscope channel, in order not to block the field of view or adversely affect camera focussing. The protruding part of the distal assembly is the only portion of the instrument that ever makes direct contact with the patient.

At the proximal end of the endoscope working channel, which is typically held 50 cm to 80 cm from the patient, the flexible shaft 112 emerges from the working channel port and extends a further 30 cm to 100 cm to the interface joint 106. In use, the interface joint 106 is typically held by a gloved assistant throughout the procedure. The interface cable 104 is connected to the generator 102 using a QMA-type coaxial interface, which is designed to allow continuous clockwise or counter clockwise rotation. This permits the interface joint 106 to rotate with the torque transfer unit 116 under the control of the user. The assistant supports the interface joint 106 throughout the procedure in order to assist the user with sympathetic instrument rotation and fluid injection.

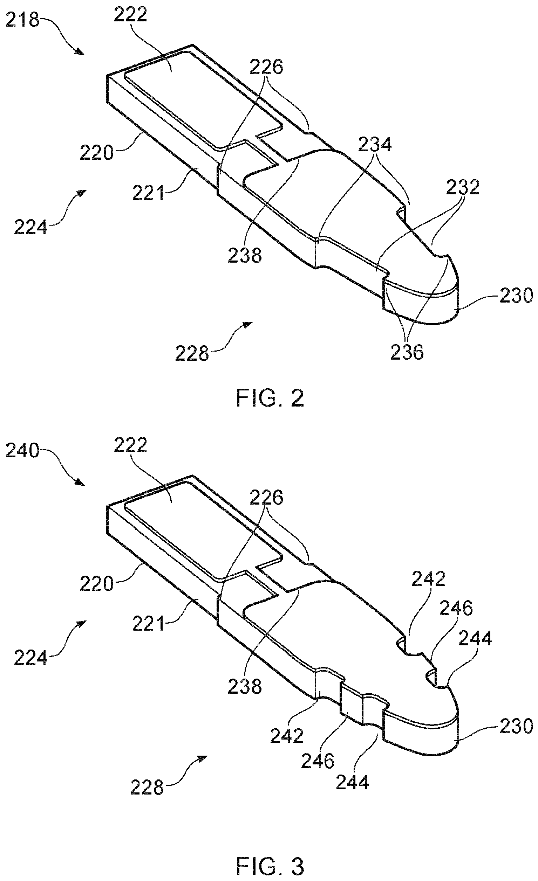

FIG 2 is a perspective view of an embodiment instrument tip 218, which may be used as the instrument tip 118 in the system of FIG 1.

The instrument tip 218 includes a planar body 220 made of a dielectric material, which separates a first conductive element 222 on a first (upper) surface of the planar body from a second conductive element (not shown) on a second (lower) surface of the planar body. A vertical edge 221 extends around the perimeter of the planar body, between the upper surface and the lower surface.

A proximal portion 224 of the instrument tip 218 may be connected to a flexible shaft (e.g. flexible shaft 112 of Figure 1) which may carry a coaxial feed cable to convey RF and/or microwave energy to the first and second conductive elements. The instrument tip 218 includes a pair of lateral steps 226, one in each lateral edge of the planar body 220. The steps 226 are configured to provide an increase in lateral width of the planar body 220 from the proximal portion 224 to a distal portion 228, and are sized to accommodate a distal end of the flexible shaft, so that the flexible shaft may be seated within the proximal portion 224 and against the steps 226 to form a substantially flush connection with the exposed (distal) portion 228 of the instrument tip.

The exposed, distal portion 228 of the instrument tip generally tapers inwardly to provide a decrease in diameter from the lateral steps 226 to a rounded distal end 230. A notch 232 is formed in each lateral edge of the exposed portion, along a section of the edge 221 that is axially between a respective step 226 and the rounded tip 230.

In this embodiment, the notches 232 have corresponding (identical and opposing) configurations so that the instrument tip is symmetrical across a central axis thereof. In particular, each notch 232 defines a recess formed by a proximal wall, a distal wall, and a rear wall that extends between the proximal and distal walls. Each of the walls are smoothly contoured together, forming a smooth concave recess. Each notch 232 forms a respective proximal pointed corner 234 in the edge 221 of the instrument tip, and a respective distal pointed corner 236 in the edge 221 of the instrument tip. Each of these corners helps to provide a visual indicator of insertion depth in use. For example, each distal pointed corner 236 may be located 1 mm from the distal end 230, and each proximal pointed corner 234 may be located 2mm from the distal end 230. These can help act as a marker for a clinician to gauge axial insertion depth into biological tissue.

The first conductive element 222 may also be configured to provide a marker of insertion depth. For example, as shown in Figure 2, the first conductive element 222 may be patterned to form an edge 238 that extends laterally across the planar body, perpendicular to a central axis thereof. This edge may be located, for example, at a distance of approximately 4.3 mm from the distal end 230.

In the embodiment of FIG 2, each notch 232 is elongate, being axially longer than it is laterally wide. Further, each of the pointed corners 234 and 236 forms a substantially perpendicular angle into the edge 221 of the instrument tip. These features may each help to capture vessels and to facilitate ease of cleaning.

FIG 3 is a perspective view of an alternative instrument tip 240 according to another embodiment of the invention. The instrument tip 240 is generally similar to the instrument tip 218, with similar reference numerals denoting similar elements, except where discussed otherwise.

The instrument tip 240 differs from the instrument tip 218 in the configuration of the notches. Whereas the instrument tip 218 includes a single elongate notch 232 on each lateral edge thereof, the instrument tip 240 includes a plurality (e.g. two) short notches 242 and 244 on each lateral edge thereof. Each lateral edge of the instrument tip 240 includes a respective proximal notch 242 and a distal notch 244. The notches each define a respective concave recess that terminates in respective distal and proximal pointed corners.

Each short notch may be substantially as (axially) long as it is (laterally) deep. Therefore, it may have a higher curvature than the elongate notches 232 of FIG 2. This higher curvature may assist with insertion depth perception, by facilitating the ease with which the midpoint of each notch may be gauged. Further, each proximal notch 242 is separated from the respective distal notch 244 by an intermediate edge portion 246, which can also provide a further indicator of depth perception. The intermediate edge portions 246 may be substantially planar, thereby not gripping or snagging tissue.

By providing a plurality of notches separated by a respective edge portion along each lateral edge, the instrument tip 240 may help to provide a finer scale of depth insertion perception compared to the single elongate notches 242 of FIG 1. For example, a distal corner of each distal notch 244 may be approximately 1 mm from the rounded end 230, a centre point of each intermediate edge portion 246 may be approximately 1.5 mm from the rounded end, and a proximal corner of each proximal notch 242 may be approximately 2 mm from the rounded end. Different reference depths may be useful for different surgical applications. For example, a 1mm guide may be useful for applications in the lower GI tract, whereas a 2mm guide may be useful for applications in the stomach.

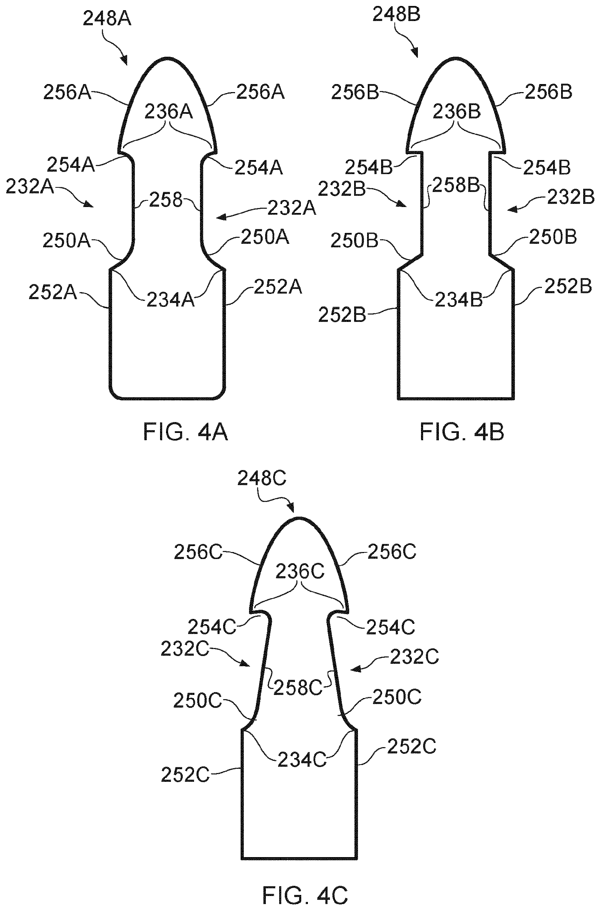

FIGs 4A to 10C show top-down views of instrument tips 248A-V having various notch configurations. For simplicity, FIGs 4A to 10C do not show the full arrangement of each instrument tip including the conductive elements, and instead show an outline of the general shape of the instrument tip. However, it will be understood that the shapes shown could be provided by one or more of the planar body, conductive element(s), and/or a protective hull (if present) on the undersurface of the planar body. Further, these instrument tips 248A-V could each be used as a modification to the instrument tips 218 of FIGs 2 and 3.

FIGs 4A to 4C show instrument tips 248A-C that each have elongate notches. The instrument tips 248A-C of FIGs 4A to 4C differ from one another primarily in the angles formed by and within the elongate notches.

For example, FIG 4A shows a top-down view of an instrument tip 248A which has a similar shape to that shown in FIG 2, with similar reference numerals denoting similar elements, except where discussed otherwise. In contrast to the instrument tip of FIG. 2, the instrument tip 248A of FIG 4A omits the lateral steps 226. However, similarly to the instrument tip 218 of FIG. 2, the instrument tip 248A has a pair of elongate notches 232A along opposing lateral sides. Each elongate notch 232A terminates in a respective proximal pointed corner 234A and distal pointed corner 236A. Each proximal pointed corner 234A forms an obtuse angle between two adjoining surfaces of the instrument tip, i.e. between a proximal end wall 250A of the notch 232A and a proximal edge portion 252A of the instrument tip (e.g. planar body) adjacent to the notch 232A. Likewise, each distal pointed corner 236A forms an obtuse angle between a distal end wall 254A of the notch and a distal edge (also referred to as a distal edge portion) 256A of the planar instrument tip (e.g. planar body) adjacent to the notch 232A. Each notch 232A includes a substantially planar rear wall 258A which is located laterally inwardly of the end walls 254A and 250A, and is smoothly contoured to the respective end walls 254A and 250A. This arrangement helps to provide a notch which mitigates clogging and can be easily cleaned.

In contrast, FIG 4B shows an arrangement having a pair of opposing elongate notches 232B which each have a distal pointed corner 254B that forms a substantially perpendicular angle between the distal end wall 254B of the notch 232B and the distal edge 256B of the instrument tip 248B. This may help to further improve traction and catch tissue within the recess of the notch. Further, within each notch 232B, the rear wall 258B and end walls 250B meet to form pointed corners, rather than forming a smoothly contoured recess as shown in FIG 4A. Similarly to FIG 4A, each proximal pointed corner 234B in FIG 4B still forms an obtuse angle in the edge of the instrument tip, which facilitates smooth insertion into tissue in a distal direction.

FIG 4C shows an arrangement having a pair of opposing elongate notches 232C which each have a distal pointed corner 236C that forms an acute angle between a distal end wall 254C of the notch and a distal edge portion 256C of the planar body. Each acute corner 236C is pointed in a proximal direction, thereby acting as a barb which can help to anchor the instrument tip in a particular position by resisting proximal movement/slipping relative to biological tissue. Each acute corner 236C helps to form/overhang a hooked recess at the distal end wall 254C of the notch 232C, further helping to snag on individual tissue fibres or vessels. The hooked recess of the notch is smoothly contoured and tapers in its lateral depth so that a proximal end of the notch is shallower than a distal end of the notch. Thus, each notch 232C terminates at its proximal end in a proximal pointed corner 234C which is at a greater obtuse angle than the proximal pointed corners 234A-B shown in FIGs 4A-4B, thereby even further facilitating smooth distal insertion into tissue.

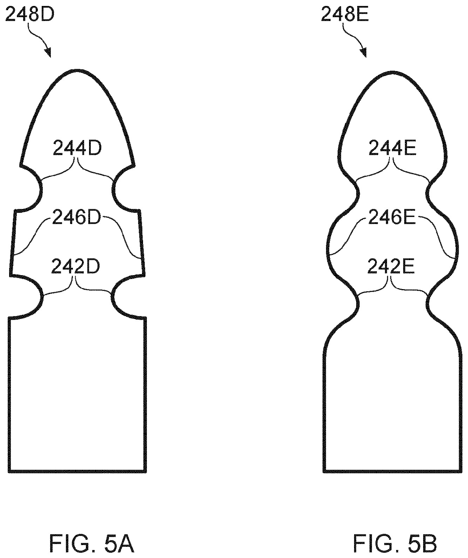

FIGs 5A and 5B show instrument tips 248D-E that each have a plurality of short notches. The instrument tip 248D of FIG 5A is similar to that shown in FIG 3, with similar reference numerals denoting similar elements. However, the instrument tip 248D of FIG 5A is modified to omit the lateral steps 226 that are shown in FIG 3. The instrument tip 248D includes a plurality (e.g. two) short notches 242D, 244D on each lateral side, with each notch terminating in a pair of pointed corners.

FIG 5B shows a further modified instrument tip 248E, whereby the notches 242E and 244E are smoothly contoured into the lateral edges of the instrument tip (e.g. planar body) rather than forming pointed corners in the edge of the instrument tip (e.g. planar body). Consequently, each intermediate edge portion 246E is likewise smoothly contoured and forms a curve between respective notches 242E and 244E.

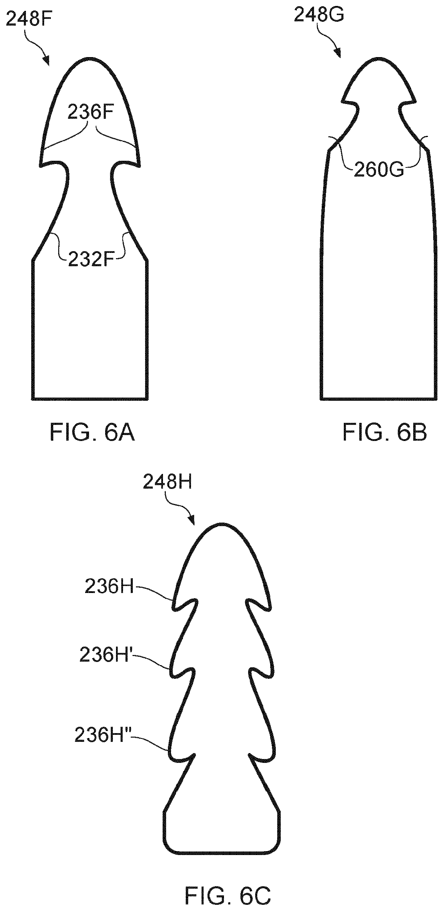

FIGs 6A to 6C show embodiment instrument tips 248F-H which may each be particularly useful for snagging and pulling on individual fibres. FIG. 6A shows an instrument tip 248F having a pair of opposing elongate notches 232F which each terminate in a barb 236F overhanging a distal end of the recess, thereby forming a hook. The instrument tip 248F is similar to the instrument tip 248C, except that the notches 232F form deeper hooked recesses which enable more snagging and help to further prevent the device from slipping.

FIG. 6B shows a variant instrument tip 248G having a pair of opposing, relatively short notches 260G which are located at (e.g. only at) the distalmost end of the instrument tip 248G, for example at a distal quarter of an exposed portion of the instrument tip 248G. In contrast, the instrument tips 248A-F had notches located along a central region thereof (e.g. within a central two quarters along the length of the exposed portion of the instrument tips). Accordingly, the instrument tip 248G is particularly useful for detailed snagging and pulling nearer the end of the instrument tip 248G.

FIG. 6C shows a variant instrument tip 248H having a plurality (e.g. three) pairs of opposing notches which are formed along a majority of the lateral edges of the instrument tip. Each notch includes a barbed distal corner 236H, 236H', and 236H". The barbed corners vary in sharpness, with the distalmost barbed corner 236H being the sharpest, and the most proximal barbed corner 236H" being the dullest.

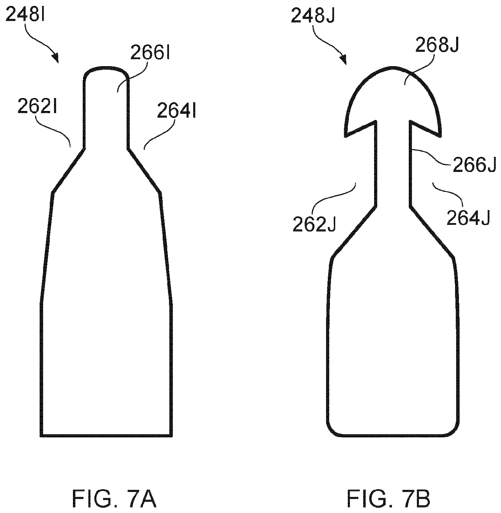

FIGs 7A-7B show variant instrument tips 248I-248J which may be particularly advantageous for detailed work. The instrument tip 248I comprises a pair of notches 262I and 264I which together form an elongate thinned section 266I of the instrument tip. The thinned section 266I has a lateral width that is approximately 30% of the maximum lateral width of the instrument tip 248I. The elongate thinned section 266I terminates in a rounded end, forming a 'pen tip' arrangement for precise control of cutting at the distal end of the instrument tip 248I.

The instrument tip 248J similarly has a pair of notches 262J and 264J which form an elongate thinned section 266J. However, the elongate thinned section 266J terminates in an enlarged section 268J which defines a pair of pointed corners that form respective barbs at the distal end of the notches 262J and 264J. The thinned section 266J maximises the size of the hook formed by the notches 262J and 264J, thereby increasing the amount of tissue that can be caught within the notches.

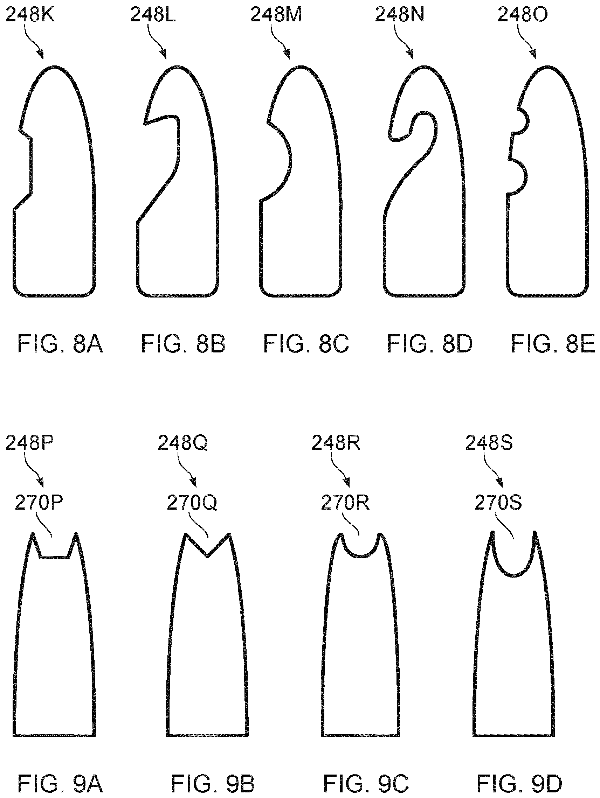

FIGs 8A to 8E show variant instrument tips 248K-O which have a different notch configuration on a first lateral side compared to a second lateral side. Specifically, the instrument tips 248K-O are each one-sided instrument tips that have notches on only a first lateral side thereof. This allows for different types of cuts to be performed depending on the orientation of the device. Further, this also allows the notch to be made larger, increasing the amount of tissue that can be caught, for example as can be seen from FIGs 8B and 8D.

FIGs 9A to 9D show variant instrument tips 248P-S which are particularly suited for delivering energy end-on to a vessel/tissue. Each instrument tip 248P-S has a single 'fish-mouth' notch 270P-S that is located at a distal end of the respective instrument tip 248P-S and faces distally away from the respective instrument tip 248P-S. Each fish mouth notch forms a pair of pointed corners that define a recess for engaging tissue therebetween. Each corner faces distally away from the distal end of the instrument tip to impinge, in use, upon tissue. The notches 270P-S each have different internal shapes to provide various contact against tissue. For example, the notch 270P forms a substantially trapezoidal recess, the notch 270Q forms a triangular recess, and the notches 270R and 270S each form rounded (oval) recesses with different depths.

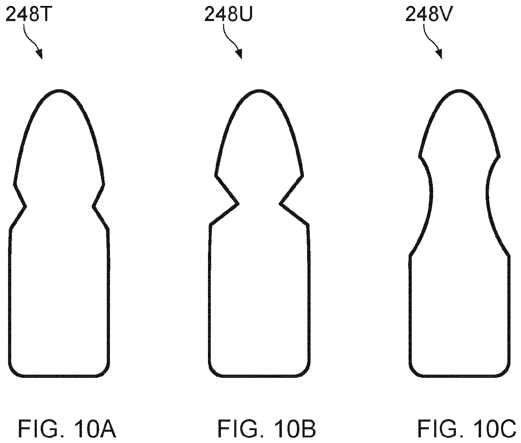

FIGs 10A to 10C show variant instrument tips 248T-V which have relatively shallow and symmetrical notches. These provide improved traction compared to an instrument tip that does not have any notches, without adding excessive friction. The notches each have a lateral depth of less than 30% of the maximum width of their respective instrument tips 248T-V.

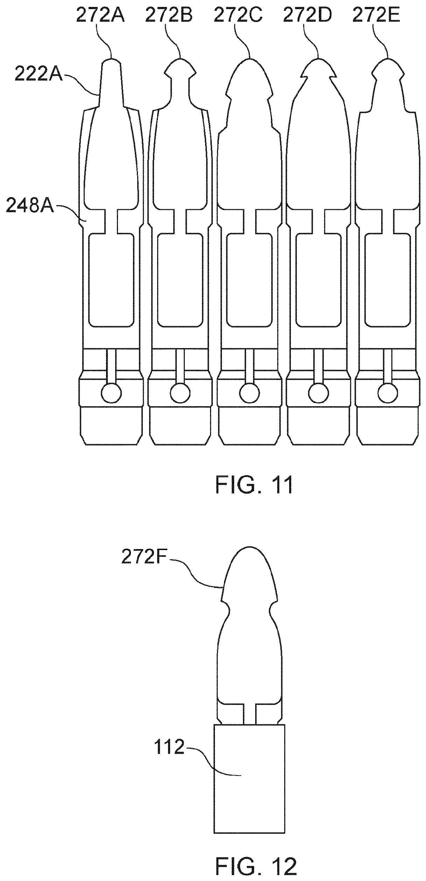

FIG 11 shows various instrument tips 272A-E having shapes similarly to those described above, but showing the detail of the planar bodies and first conductive elements. For example, the instrument tip 272A has a planar body 248A which has a pen-tip notch configuration similar to that shown in FIG 7A, where the conductive element 222A does not include any notches formed therein. In contrast, the instrument tips 272B-E each have notches formed in both their planar bodies and their first conductive elements.

FIG 12 shows yet a further instrument tip 272F, but which is connected to a flexible shaft 112. The instrument tip 272F includes a pair of opposing notches, each notch having a pointed distal end and a smoothly contoured proximal end. This provides increased tissue engagement at the distal edge of the notch, while also ensuring that the proximal end of the notch does not inhibit insertion of the instrument tip. The flexible shaft 112 is substantially flush with the exposed portion of the instrument tip 272F, since it is seated against a pair of lateral steps formed in the planar body of the instrument tip 272F, similarly to those described above in relation to FIGs 2 and 3.

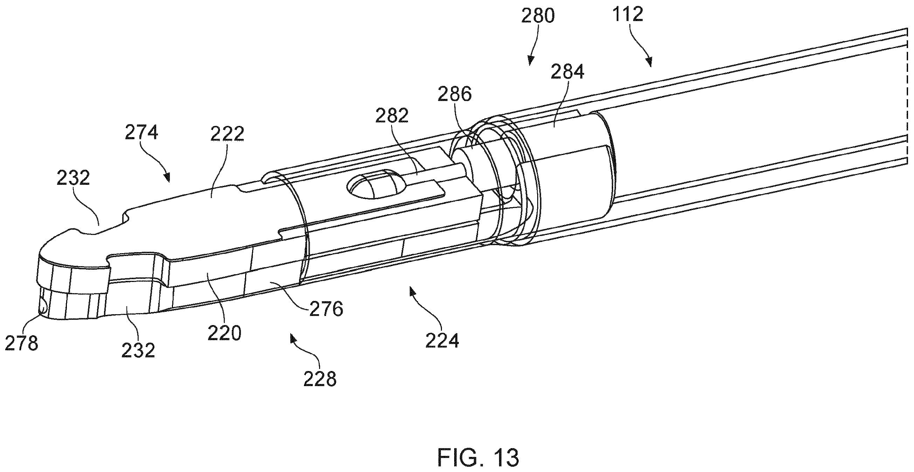

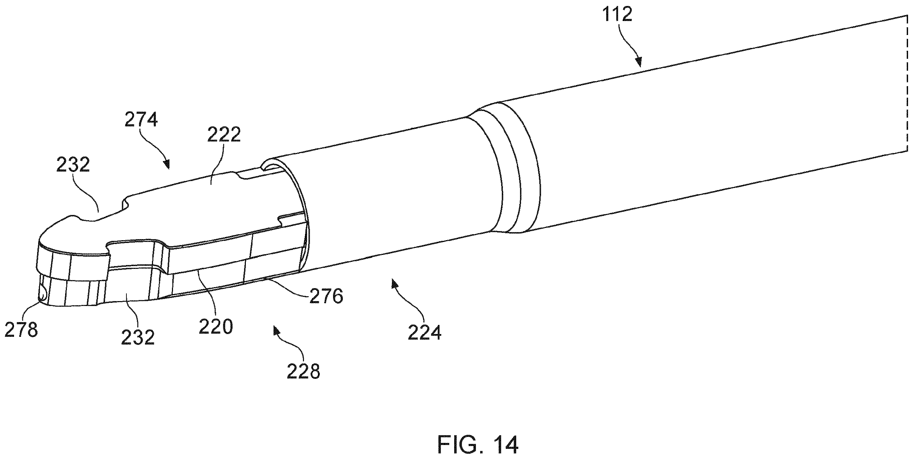

FIGs 13 and 14 show a variant instrument tip 274 connected to a flexible shaft.112, e.g. for use in the system of FIG 1. The instrument tip 274 is similar to the instrument tip 218 of FIG. 2, in that they both have a pair of opposing elongate notches.

Similarly to FIG. 2, the instrument tip 274 comprises an active tip which includes a planar body 220 separating a first conductive element 222 from a second conductive element. A pair of elongate notches 232 are formed in opposing lateral edges of the active tip. In FIGs 13 and 14, the instrument tip 274 further includes a protective hull 276 positioned under the active tip. The protective hull 276 comprises a piece of dielectric material, which may have a smoothly contoured convex undersurface facing away from the planar body 220. Optionally, the protective hull 276 may include a channel (e.g. tubular channel or needle) 278 for conveying fluid into a treatment zone. As shown in FIGs 13 and 14, the notches 232 may be formed in opposing lateral edges of the protective hull 276 as well as in the active tip.

The instrument tip 274 of FIGs 13 and 14 further differs from the instrument tip 218 of FIG 2 in that the first conductive element 222 of the instrument tip 274 has a different pattern that covers a different (e.g. larger) proportion of the planar body 220 compared to the first conductive element 222 of the instrument tip 218. The second conductive element may have a similar pattern to the first conductive element 222 in each embodiment.

FIGs 13 and 14 further show the connection between the flexible shaft 112 and the instrument tip 274. In FIG 13, the shaft 112 is transparent for illustrative purposes, to show its electrical connection with the instrument tip 274. In particular, the flexible shaft 112 comprises a coaxial cable 280 for conveying an RF and/or microwave frequency EM signal to the instrument tip 274. The coaxial cable 280 comprises an inner conductor 282, an outer conductor 284 coaxial with the inner conductor 282, and a dielectric material 286 separating the inner and outer conductors. The inner conductor 282 is electrically connected to the first conductive element 222 and the outer conductor 284 is electrically connected to the second conductive element to enable the instrument tip to receive the EM signal.

It will be understood that any variant instrument tip described herein could alternatively be connected to the coaxial feed cable in a corresponding manner. The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the appended claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Reference numerals

- 100: electrosurgery system
102 generator
104 interface cable
106 interface joint
108 fluid delivery apparatus electrosurgical instrument
112 flexible shaft
280 coaxial cable
282 inner conductor
284 outer conductor
286 dielectric material
118, 218, 240, 248A-V, 272A-F, 274 instrument tip
220, 248 planar body
222 first conductive element
238 edge
276 protective hull
278 channel
224 proximal portion
226 lateral steps
228 distal portion
230 distal end
232 elongate notch
234 proximal pointed corner
236 distal pointed corner
250 proximal end wall
254 distal end wall
258 rear wall
242 proximal notch
244 distal notch
246 intermediate edge portion
252 proximal edge portion
256 distal edge portion
266 elongate thinned section
262, 264 pair of notches
268 enlarged section
270 fish mouth notch
107 fluid supply cable
114 surgical scoping device
116 torque transfer unit

## Claims

1. An electrosurgical instrument for applying radiofrequency, RF, electromagnetic, EM, energy and/or microwave frequency EM energy to biological tissue, the electrosurgical instrument comprising:
an instrument tip (274) comprising a planar body (220) made of a first dielectric material separating a first conductive element (222) on an upper surface thereof from a second conductive element on a lower surface thereof, the lower surface facing in the opposite direction to the upper surface;
a coaxial feed cable (280) comprising an inner conductor (282), an outer conductor (284) coaxial with the inner conductor and a dielectric material (286) separating the inner and outer conductors, the coaxial feed cable being for conveying an RF signal and/or microwave signal;
wherein the inner conductor is electrically connected to the first conductive element and the outer conductor is electrically connected to the second conductive element to enable the instrument tip to receive the RF signal and/or the microwave signal;
**characterised in that** an edge of the planar body has a notch (232) defining a recess for engaging tissue-within the recess.

2. The electrosurgical instrument of claim 1, wherein at least one end of the notch forms a pointed corner (234C, 236C) in the edge of the planar body.

3. The electrosurgical instrument of claim 2, wherein the pointed corner (234C) forms an obtuse angle between two adjoining surfaces of the edge of the planar body.

4. The electrosurgical instrument of claim 2, wherein the pointed corner (236C) forms an acute angle between two adjoining surfaces of the edge of the planar body, and
optionally wherein the pointed corner comprises a barb pointing in a proximal direction.

5. The electrosurgical instrument of any one of claims 2 to 4, wherein a distal end of the notch forms the pointed corner (236C).

6. The electrosurgical instrument of any preceding claim, wherein at least one end of the notch (232A) is smoothly contoured into the edge of the planar body, and
optionally wherein a proximal end of the notch is smoothly contoured into the edge of the planar body.

7. The electrosurgical instrument of any preceding claim, wherein the notch is located at a distal end (230) of the planar body and faces distally away from the instrument tip, and
optionally wherein the notch (252D, 244D) forms a pair of pointed corners defining the recess for engaging tissue therebetween, each corner facing away from the distal end of the planar body to impinge upon the tissue.

8. The electrosurgical instrument of any one of claims 1 to 6, wherein the notch is located at a lateral edge of the planar body, and
optionally wherein the notch is an elongate notch located along the lateral edge, and
optionally wherein the notch has a lateral depth of less than 30% of the maximum width of the instrument tip, optionally less than 20%, optionally less than 15%.

9. The electrosurgical instrument of any preceding claim, comprising two or more notches, and
optionally wherein the two or more notches are spaced apart in an axial direction.

10. The electrosurgical instrument of claim 14, wherein the two or more notches are located at opposite lateral edges of the planar body.

11. The electrosurgical instrument of claim 10, wherein the two or more notches together form an elongate thinned section (266I) of the instrument tip, the thinned section having a width of less than 50% of the maximum width of the instrument tip, optionally less than 40%, optionally less than 30%, optionally less than 20%.

12. The electrosurgical instrument of claim 11, wherein one of the following applies:
(a) the elongate thinned section terminates in a rounded end,
(b) the elongate thinned section terminates in an enlarged section (268J) to define a pointed corner.

13. The electrosurgical instrument of any preceding claim, wherein the planar body has the same notch configuration on both lateral sides thereof.

14. The electrosurgical instrument of any one of claims 1 to 12, wherein the instrument tip has a different notch configuration on a first lateral side of the instrument tip than on a second lateral side of the instrument tip.

15. The electrosurgical instrument of any preceding claim, wherein at least one of the following applies:
(a) the first conductive element is patterned to provide an indication of axial and/or lateral depth,
(b) the first and/or second conductive elements extend up to a distal end of the planar body.

## Patentansprüche

1. Elektrochirurgisches Instrument zum Beaufschlagen von Hochfrequenz-, HF-, elektromagnetischer, EM-, Energie und/oder Mikrowellenfrequenz-EM-Energie auf biologisches Gewebe, wobei das elektrochirurgische Instrument Folgendes umfasst:
eine Instrumentenspitze (274), umfassend einen planaren Körper (220) aus einem ersten dielektrischen Material, das ein erstes leitfähiges Element (222) auf einer Oberseite desselben von einem zweiten leitfähigen Element auf einer Unterseite desselben trennt, wobei die Unterseite in die zur Oberseite entgegengesetzte Richtung gewandt ist;
ein koaxiales Zufuhrkabel (280), umfassend einen Innenleiter (282), einen Außenleiter (284), der koaxial mit dem Innenleiter ist, und ein dielektrisches Material (286), das den Innen- und den Außenleiter voneinander trennt, umfasst, wobei das koaxiale Zufuhrkabel dem Übertragen eines HF-Signals und/oder eines Mikrowellensignals dient;
wobei der Innenleiter elektrisch mit dem ersten leitfähigen Element verbunden ist und der Außenleiter elektrisch mit dem zweiten leitfähigen Element verbunden ist, um zu ermöglichen, dass die Instrumentenspitze das HF-Signal und/oder das Mikrowellensignal empfängt;
**dadurch gekennzeichnet, dass** eine Kante des planaren Körpers eine Einbuchtung (232) aufweist, die eine Vertiefung zum Ineingriffbringen von Gewebe mit der Vertiefung definiert.

2. Elektrochirurgisches Instrument nach Anspruch 1, wobei zumindest ein Ende der Einbuchtung eine spitze Ecke (234C, 236C) in der Kante des planaren Körpers ausbildet.

3. Elektrochirurgisches Instrument nach Anspruch 2, wobei die spitze Ecke (234C) einen stumpfen Winkel zwischen zwei benachbarten Flächen der Kante des planaren Körpers ausbildet.

4. Elektrochirurgisches Instrument nach Anspruch 2, wobei die spitze Ecke (236C) einen spitzen Winkel zwischen zwei benachbarten Flächen der Kante des planaren Körpers ausbildet und
wobei gegebenenfalls die spitze Ecke einen in proximale Richtung gerichteten Widerhaken umfasst.

5. Elektrochirurgisches Instrument nach einem der Ansprüche 2 bis 4, wobei ein distales Ende der Einbuchtung die spitze Ecke (236C) ausbildet.

6. Elektrochirurgisches Instrument nach einem der vorangegangenen Ansprüche, wobei zumindest ein Ende der Einbuchtung (232A) nahtlos in die Kante des planaren Körpers übergeht und
wobei gegebenenfalls ein proximales Ende der Einbuchtung nahtlos in die Kante des planaren Körpers übergeht.

7. Elektrochirurgisches Instrument nach einem der vorangegangenen Ansprüche, wobei die Einbuchtung an einem distalen Ende (230) des planaren Körpers angeordnet ist und distal von der Instrumentenspitze weggewandt ist und
wobei gegebenenfalls die Einbuchtung (252D, 244D) ein Paar von spitzen Ecken ausbildet, das die Vertiefung zum Ineingriffbringen von Gewebe dazwischen definiert, wobei jede Ecke von dem distalen Ende des planaren Körpers weggewandt ist, um das Gewebe zu beaufschlagen.

8. Elektrochirurgisches Instrument nach einem der Ansprüche 1 bis 6, wobei die Einbuchtung an einer Seitenkante des planaren Körpers angeordnet ist und
wobei gegebenenfalls die Einbuchtung eine längliche Einbuchtung ist, die entlang der Seitenkante angeordnet ist, und
wobei gegebenenfalls die Einbuchtung eine seitliche Tiefe von weniger als 30 % der Maximalbreite der Instrumentenspitze aufweist, gegebenenfalls weniger als 20 %, gegebenenfalls weniger als 15 %.

9. Elektrochirurgisches Instrument nach einem der vorangegangenen Ansprüche, umfassend zwei oder mehrere Einbuchtungen und
wobei gegebenenfalls die zwei oder mehreren Einbuchtungen in Achsrichtung beabstandet sind.

10. Elektrochirurgisches Instrument nach Anspruch 14, wobei die zwei oder mehreren Einbuchtungen an entgegengesetzten Seitenkanten des planaren Körpers angeordnet sind.

11. Elektrochirurgisches Instrument nach Anspruch 10, wobei die zwei oder mehreren Einbuchtungen zusammen einen länglichen verdünnten Abschnitt (266I) der Instrumentenspitze ausbilden, wobei der verdünnte Abschnitt eine Breite von weniger als 50 % der Maximalbreite der Instrumentenspitze aufweist, gegebenenfalls weniger als 40 %, gegebenenfalls weniger als 30 %, gegebenenfalls weniger als 20 %.

12. Elektrochirurgisches Instrument nach Anspruch 11, wobei eines aus folgenden zutrifft:
(a) der längliche verdünnte Abschnitt endet in einem abgerundeten Ende,
(b) der längliche verdünnte Abschnitt endet in einem vergrößerten Abschnitt (268J), um eine spitze Ecke zu definieren.

13. Elektrochirurgisches Instrument nach einem der vorangegangenen Ansprüche, wobei der planare Körper auf beiden Seiten desselben die gleiche Einbuchtungskonfiguration aufweist.

14. Elektrochirurgisches Instrument nach einem der Ansprüche 1 bis 12, wobei die Instrumentenspitze auf einer ersten lateralen Seite der Instrumentenspitze eine andere Einbuchtungskonfiguration als auf einer zweiten lateralen Seite der Instrumentenspitze aufweist.

15. Elektrochirurgisches Instrument nach einem der vorangegangenen Ansprüche, wobei zumindest eines aus folgenden zutrifft:
(a) das erste leitfähige Element ist strukturiert, um eine Angabe von axialer und/oder seitlicher Tiefe bereitzustellen,
(b) das erste und/oder das zweite leitfähige Element erstreckt/erstrecken sich bis zu einem distalen Ende des planaren Körpers.

## Revendications

1. Instrument électrochirurgical pour appliquer une énergie électromagnétique, EM, radiofréquence, RF, et/ou une énergie EM hyperfréquence à un tissu biologique, l'instrument électrochirurgical comprenant :
une pointe d'instrument (274) comprenant un corps plan (220) constitué d'un premier matériau diélectrique séparant un premier élément conducteur (222) sur une surface supérieure de celui-ci d'un second élément conducteur sur une surface inférieure de celui-ci, la surface inférieure étant orientée dans la direction opposée à la surface supérieure ;
un câble d'alimentation coaxial (280) comprenant un conducteur interne (282), un conducteur externe (284) coaxial au conducteur interne et un matériau diélectrique (286) séparant les conducteurs interne et externe, le câble d'alimentation coaxial étant destiné à transporter un signal RF et/ou un signal hyperfréquence ;
dans lequel le conducteur interne est connecté électriquement au premier élément conducteur et le conducteur externe est connecté électriquement au second élément conducteur afin de permettre à la pointe d'instrument de recevoir le signal RF et/ou le signal hyperfréquence ;
**caractérisé en ce qu'**un bord du corps plan présente une encoche (232) définissant un évidement pour une mise en prise de tissu à l'intérieur de l'évidement.

2. Instrument électrochirurgical selon la revendication 1, dans lequel au moins une extrémité de l'encoche forme un coin pointu (234C, 236C) dans le bord du corps plan.

3. Instrument électrochirurgical selon la revendication 2, dans lequel le coin pointu (234C) forme un angle obtus entre deux surfaces adjacentes du bord du corps plan.

4. Instrument électrochirurgical selon la revendication 2, dans lequel le coin pointu (236C) forme un angle aigu entre deux surfaces adjacentes du bord du corps plan, et
facultativement dans lequel le coin pointu comprend un ergot pointant dans une direction proximale.

5. Instrument électrochirurgical selon l'une quelconque des revendications 2 à 4, dans lequel une extrémité distale de l'encoche forme le coin pointu (236C).

6. Instrument électrochirurgical selon l'une quelconque des revendications précédentes, dans lequel au moins une extrémité de l'encoche (232A) est profilée de manière lisse dans le bord du corps plan, et
facultativement dans lequel une extrémité proximale de l'encoche est profilée de manière lisse dans le bord du corps plan.

7. Instrument électrochirurgical selon l'une quelconque des revendications précédentes, dans lequel l'encoche est située à une extrémité distale (230) du corps plan et est orientée distalement à l'opposé de la pointe d'instrument, et
facultativement dans lequel l'encoche (252D, 244D) forme une paire de coins pointus définissant l'évidement pour une mise en prise de tissu entre ceux-ci, chaque coin étant orienté à l'opposé de l'extrémité distale du corps plan pour heurter le tissu.

8. Instrument électrochirurgical selon l'une quelconque des revendications 1 à 6, dans lequel l'encoche est située au niveau d'un bord latéral du corps plan, et
facultativement dans lequel l'encoche est une encoche allongée située le long du bord latéral, et facultativement dans lequel l'encoche présente une profondeur latérale inférieure à 30 % de la largeur maximale de la pointe d'instrument, facultativement inférieure à 20 %, facultativement inférieure à 15 %.

9. Instrument électrochirurgical selon l'une quelconque des revendications précédentes, comprenant deux encoches ou plus, et
facultativement dans lequel les deux encoches ou plus sont espacées dans une direction axiale.

10. Instrument électrochirurgical selon la revendication 14, dans lequel les deux encoches ou plus sont situées au niveau de bords latéraux opposés du corps plan.

11. Instrument électrochirurgical selon la revendication 10, dans lequel les deux encoches ou plus forment ensemble une section amincie allongée (266I) de la pointe d'instrument, la section amincie présentant une largeur inférieure à 50 % de la largeur maximale de la pointe d'instrument, facultativement inférieure à 40 %, facultativement inférieure à 30 %, facultativement inférieure à 20 %.

12. Instrument électrochirurgical selon la revendication 11, dans lequel l'un des points suivants s'applique :
(a) la section amincie allongée se termine par une extrémité arrondie,
(b) la section amincie allongée se termine par une section élargie (268J) pour définir un coin pointu.

13. Instrument électrochirurgical selon l'une quelconque des revendications précédentes, dans lequel le corps plan présente la même configuration d'encoche sur les deux côtés latéraux de celui-ci.

14. Instrument électrochirurgical selon l'une quelconque des revendications 1 à 12, dans lequel la pointe d'instrument présente une configuration d'encoche différente sur un premier côté latéral de la pointe d'instrument que sur un second côté latéral de la pointe d'instrument.

15. Instrument électrochirurgical selon l'une quelconque des revendications précédentes, dans lequel au moins l'un des points suivants s'applique :
(a) le premier élément conducteur est structuré pour fournir une indication de profondeur axiale et/ou latérale,
(b) les premier et/ou second éléments conducteurs s'étendent jusqu'à une extrémité distale du corps plan.
